# EUROPEAN PATENT SPECIFICATION

(11) **EP 4 082 338 B1**
(45) Date of publication and mention of the grant of the patent: **14.08.2024**
(21) Application number: 21796565.6
(22) Date of filing: 20.02.2021
(51) Int. Cl.: A01N 1/02, G01N 1/28, C12Q 1/24, G01N 1/30

(54) **ALCOHOL-BASED JELLY COMPOSITION FOR CELL FIXATION**
GELZUSAMMENSETZUNG AUF ALKOHOLBASIS ZUR ZELLFIXIERUNG
COMPOSITION DE GELÉE À BASE D'ALCOOL POUR FIXATION CELLULAIRE

(30) Priority: 29.04.2020 KR 20200052920
(43) Date of publication of application: 02.11.2022
(73) Proprietor: Biodyne Co., Ltd., Seongdong-gu Seoul 04793 (KR)
(72) Inventor: IM, Wook Bin, Seoul 06280 (KR)
(74) Representative: Hoefer & Partner Patentanwälte mbB
(86) International application number: PCT/KR2021/002159
(87) International publication number: WO 2021/221279

(56) References cited:
- EP-A2- 0 511 430
- WO-A1-01/70022
- WO-A1-2011/146909
- WO-A2-2017/083729
- JP-A- 2010 518 870
- KR-A- 20050 116 689
- KR-B1- 102 182 077
- US-A- 6 051 425
- US-A1- 2006 088 814

## Description

### Field of the Invention

The present invention relates to a technology for stably maintaining a specimen (for example, exfoliative cells of a human body) contained in a vial so that the specimen can be safely transported over a long distance.

More specifically, the present invention is a technology for preventing deformation of a specimen in a vial by converting a liquid for specimen preservation contained in the vial into a jelly state and thus effectively preventing leakage of the liquid.

### Background Art

In general, the state of exfoliative cells can be observed for diagnosis after a specimen (exfoliative cells) is obtained from the human body and then the exfoliative cells are spread on the slide for cytodiagnosis at the examination center.

Here, patients who have difficulty visiting the examination center should collect their own exfoliative cells (specimen) and then have the exfoliative cells transported to the examination center in a state in which the exfoliative cells are intact. To this end, the specimen may be put in a vial, which is an airtight container, together with the liquid for cell fixation to protect the specimen and transported to the examination center.

The liquid for cell fixation filled in the vial to prevent specimen deformation is generally composed of an aqueous solution. In the process of transporting the vial, the liquid for cell fixation frequently leaks through the gap between the body and the cap of the vial, and this may cause deformation of the specimen.

It is required to develop a technology which may be capable of solving such problems of the prior art.

Meanwhile, the prior literatures related to the present invention are as follows.
(1) EP 0511430 A2 (November 04, 1992) "Cell preservative solution"
(2) US 2006/0088814 A1 (April 27, 2006) "Enhanced cell preservative solution and methods for using same"
(3) US 6,632,666 B2 (October 14, 2003) "Normothermic, hypothermic and cryopreservation maintenance and storage of cells, tissues and organs in cell-based media"
(4) JP 2010-518870 A (June 03, 2010) "Fixation of a biological material"

### DISCLOSURE OF INVENTION

### Technical Problem

The present invention has been proposed in view of the above points, and an object thereof is to provide an alcohol-based jelly composition for cell fixation capable of stably maintaining a specimen contained in a vial so that the specimen can be safely transported over a long distance.

### Technical Solution

In order to achieve the object, the alcohol-based jelly composition for cell fixation according to the present invention may be configured to contain 30 to 60 parts by weight of an aqueous methanol solution, 0.1 to 0.2 parts by weight of EDTA, 0.01 to 0.05 parts by weight of cholic acid, 0.05 to 0.1 parts by weight of a 1 N aqueous acetic acid solution, and 5 to 20 parts by weight of any one or more of gelatin or a photocurable protein (gelatin methacryloly).

The alcohol-based jelly composition for cell fixation according to the present invention may be configured to contain 40 to 50 parts by weight of an aqueous ethanol solution, 0.1 to 0.2 parts by weight of EDTA, 0.2 to 1 part by weight of a dihydric alcohol additive, and 5 to 20 parts by weight of one or more of gelatin or a photocurable protein.

The alcohol-based jelly composition for cell fixation according to the present invention may be configured to contain 30 to 60 parts by weight of an aqueous methanol solution, 0.1 to 0.2 parts by weight of EDTA, 0.01 to 0.05 parts by weight of cholic acid, 0.05 to 0.1 parts by weight of a 1 N aqueous acetic acid solution, and 1 to 100 parts by weight of a natural cellulose polymer.

The alcohol-based jelly composition for cell fixation according to the present invention may be configured to contain 40 to 50 parts by weight of an aqueous ethanol solution, 0.1 to 0.2 parts by weight of EDTA, 0.2 to 1 part by weight of a dihydric alcohol additive, and 1 to 100 parts by weight of a natural cellulose polymer.

The alcohol-based jelly composition for cell fixation according to the present invention may be configured to contain 30 to 60 parts by weight of an aqueous methanol solution, 0.1 to 0.2 parts by weight of EDTA, 0.01 to 0.05 parts by weight of cholic acid, 0.05 to 0.1 parts by weight of a 1 N aqueous acetic acid solution, and 1 to 100 parts by weight of synthesized acrylic polymer beads.

The alcohol-based jelly composition for cell fixation according to the present invention may be configured to contain 40 to 50 parts by weight of an aqueous ethanol solution, 0.1 to 0.2 parts by weight of EDTA, 0.2 to 1 part by weight of a dihydric alcohol additive, and 1 to 100 parts by weight of synthesized acrylic polymer beads.

### Advantageous Effects

The present invention has an advantage that the jellification of a liquid for cell fixation filled inside a vial is simple since the liquid for cell fixation is jellified by mixing the selected solution and the selected material.

The present invention has an advantage that the leakage of a liquid for cell fixation from a vial can be prevented during long-distance transport by the jellification of the liquid for cell fixation.

The present invention also has an advantage that the process of putting a specimen inside a vial filled with a liquid for cell fixation is simple.

### Brief Description of the Drawings

FIG. 1 is an exemplary view of a vial that contains a specimen for transport.
FIG. 2 is an exemplary view of a state in which a vial is mounted on a stirrer for jellification.
FIG. 3 is a flow chart illustrating a jellification process using an alcohol-based jelly composition for cell fixation according to a first embodiment of the present invention.
FIG. 4 is a flow chart illustrating a jellification process using an alcohol-based jelly composition for cell fixation according to a second embodiment of the present invention.

### Embodiment for Carrying Out the Invention

Hereinafter, the present invention will be described in detail.

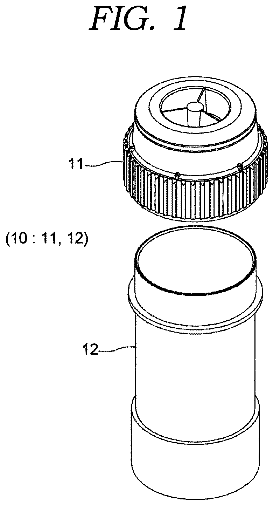

FIG. 1 is an exemplary view of a vial, which is an airtight container that contains a specimen for transport. Referring to FIG. 1, a vial 10 may include a hollow body 12 having open one end and a cap 11 opening and closing the opening portion of the body 12.

At this time, the vial 10 may be fabricated by extruding a resin containing an antioxidant since the alcohol component may be oxidized when the vial 10 is exposed to light or oxygen in a state of containing the liquid for cell fixation. The vial 10 may also be fabricated to be translucent by adding a light blocking dye.

It may be possible to prevent the exposure to oxygen or light by adopting an opaque pouch-type packaging material as the container containing the liquid for cell fixation.

Meanwhile, the specimen portion not submerged in the liquid for cell fixation may be damaged in the process of putting the specimen-collected brush tip (not illustrated) inside the vial 10. It is thus preferable that the vial 10 is fabricated in a size that can completely accommodate the brush tip. It is also preferable to fully fill the liquid for cell fixation in the vial 10 in a state in which the specimen-collected brush tip is placed inside the vial 10.

Conventionally, when the liquid for cell fixation in the form of an aqueous solution was filled in the vial 10 together with the specimen, a problem of specimen deformation due to leakage during long-distance transport has occurred. In order to solve this problem, the present invention provides a composition capable of jellifying the liquid for cell fixation. In other words, when the liquid for cell fixation is jellified, the possibility of leakage or evaporation is low, and the original state of specimen can be maintained.

The alcohol-based jelly composition for cell fixation according to the first embodiment of the present invention may be configured to contain 30 to 60 parts by weight of an aqueous methanol solution, 0.1 to 0.2 parts by weight of EDTA (ethylenediaminetetraacetic acid), 0.01 to 0.05 parts by weight of cholic acid, 0.05 to 0.1 parts by weight of a 1 N aqueous acetic acid solution, and 5 to 20 parts by weight of any one or more of gelatin or a photocurable protein (gelatin methacryloly).

The physical properties depending on the composition ratio were examined for the first embodiment of the present invention as described above, and the influence of an individual component on the physical properties of the entire composition was examined while varying the mixing ratio of the individual component.

Table 1 presents the experimental conditions in which the mixing ratio of only the aqueous methanol solution is varied while the mixing ratios of other components are fixed in the first embodiment of the present invention.

**[Table 1]**

| "Experiment 1" | Example | | | Comparative Example | |
|---|---|---|---|---|---|
| | A1 | B1 | C1 | a1 | b1 |
| Aqueous methanol solution (30-60) | 30 | 45 | 60 | 25 | 65 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |
| Gelatin (5-20) | 13 | 13 | 13 | 13 | 13 |

Table 2 presents the experimental conditions in which the mixing ratio of only EDTA is varied while the mixing ratios of other components are fixed in the first embodiment of the present invention.

**[Table 2]**

| "Experiment 2" | Example | | | Comparative Example | |
|---|---|---|---|---|---|
| | A2 | B2 | C2 | a2 | b2 |
| Aqueous methanol solution (30-60) | 45 | 45 | 45 | 45 | 45 |
| EDTA (0.1-0.2) | 0.1 | 0.15 | 0.2 | 0.05 | 0.25 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |
| Gelatin (5-20) | 13 | 13 | 13 | 13 | 13 |

Table 3 presents the experimental conditions in which the mixing ratio of only cholic acid is varied while the mixing ratios of other components are fixed in the first embodiment of the present invention.

**[Table 3]**

| "Experiment 3" | Example | | | Comparative Example | |
|---|---|---|---|---|---|
| | A3 | B3 | C3 | a3 | b3 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.02 | 0.03 | 0.04 | 0.00 | 0.07 |
| 1 N aqueous acetic acid solution (0.05-0.1) | 0.07 | 0.07 | 0.07 | 0.07 | 0.07 |
| Gelatin (5-20) | 13 | 13 | 13 | 13 | 13 |

Table 4 presents the experimental conditions in which the mixing ratio of only the 1 N aqueous acetic acid solution is varied while the mixing ratios of other components are fixed in the first embodiment of the present invention.

**[Table 4]**

| "Experiment 4" | Example | | | Comparative Example | |
|---|---|---|---|---|---|
| | A4 | B4 | C4 | a4 | b4 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.06 | 0.07 | 0.09 | 0.03 | 0.13 |
| Gelatin (5-20) | 13 | 13 | 13 | 13 | 13 |

Table 5 presents the experimental conditions in which the mixing ratio of only gelatin is varied while the mixing ratios of other components are fixed in the first embodiment of the present invention.

**[Table 5]**

| "Experiment 5" | Example | | | Comparative Example | |
|---|---|---|---|---|---|
| | A5 | B5 | C5 | a5 | b5 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |
| Gelatin (5-20) | 6 | 13 | 19 | 3 | 23 |

In Tables 1 to 5, in Examples (A1 to A5, B1 to B5, and C1 to C5), the fixing ability of the jellified cell fixing means was favorable. On the other hand, in Comparative Examples (a1 to a5 and b1 to b5), the ability to fix the specimen inside the vial 10 was too strong or too weak. Therefore, in Comparative Examples, a problem (for example, specimen deformation) occurred in the process of taking out the specimen from the vial 10 and examining (for example, redissolving) the specimen.

With regard to the first embodiment of the present invention, experiments were conducted in addition to those presented in Tables 1 to 5, but the description of the other experiments has been omitted for convenience of explanation.

Meanwhile, in the first embodiment of the present invention, 5 to 20 parts by weight of any one or more of gelatin or a photocurable protein (gelatin methacryloly) may be substituted with 1 to 100 parts by weight of a natural cellulose polymer (for example, mecellose or hecellose) or 1 to 100 parts by weight of synthesized acrylic polymer beads (for example, methyl methacrylate-co-ethylene glycol dimethacrylate; MMA).

Here, with regard to 1 to 100 parts by weight of a natural cellulose polymer as well, an experiment was conducted in mixing ratio ranges of the natural cellulose polymer of less than 1 part by weight and more than 100 parts by weight as Comparative Examples while the mixing ratios of other components were fixed as in Tables 1 to 5, and as a result, the specimen fixing ability was poor in the region outside of 1 to 100 parts by weight.

In addition, with regard to 1 to 100 parts by weight of synthesized acrylic polymer beads as well, an experiment was conducted in mixing ratio ranges of the synthesized acrylic polymer beads of less than 1 part by weight and more than 100 parts by weight as Comparative Examples while the mixing ratios of other components were fixed as in Tables 1 to 5, and as a result, the specimen fixing ability was poor in the region outside of 1 to 100 parts by weight.

The alcohol-based jelly composition for cell fixation according to the second embodiment of the present invention may be configured to contain 40 to 50 parts by weight of an aqueous ethanol solution, 0.1 to 0.2 parts by weight of EDTA, 0.2 to 1 parts by weight of a dihydric alcohol additive, and 5 to 20 parts by weight of one or more of gelatin or a photocurable protein.

The physical properties depending on the composition ratio were examined for the second embodiment of the present invention as described above, and the influence of an individual component on the physical properties of the entire composition was examined while varying the mixing ratio of the individual component.

Table 6 presents the experimental conditions in which the mixing ratio of only the aqueous ethanol solution is varied while the mixing ratios of other components are fixed in the second embodiment of the present invention.

**[Table 6]**

| "Experiment 6" | Example | | | Comparative Example | |
|---|---|---|---|---|---|
| | A6 | B6 | C6 | a6 | b6 |
| Aqueous ethanol solution (40-50) | 40 | 45 | 50 | 30 | 60 |
| EDTA (0.1-0.2) | 0.15 | 0.15 | 0.15 | 0.15 | 0.15 |
| Dihydric alcohol additive (0.2-1) | 0.6 | 0.6 | 0.6 | 0.6 | 0.6 |
| Gelatin (5-20) | 6 | 13 | 19 | 3 | 23 |

Table 7 presents the experimental conditions in which the mixing ratio of only EDTA is varied while the mixing ratios of other components are fixed in the second embodiment of the present invention.

**[Table 7]**

| "Experiment 7" | Example | | | Comparative Example | |
|---|---|---|---|---|---|
| | A7 | B7 | C7 | a7 | b7 |
| Aqueous ethanol solution (40-50) | 45 | 45 | 45 | 45 | 45 |
| EDTA (0.1-0.2) | 0.1 | 0.15 | 0.2 | 0.05 | 0.25 |
| Dihydric alcohol additive (0.2-1) | 0.6 | 0.6 | 0.6 | 0.6 | 0.6 |
| Gelatin (5-20) | 13 | 13 | 13 | 13 | 13 |

Table 8 presents the experimental conditions in which the mixing ratio of only the dihydric alcohol additive is varied while the mixing ratios of other components are fixed in the second embodiment of the present invention.

**[Table 8]**

| "Experiment 8" | Example | | | Comparative Example | |
|---|---|---|---|---|---|
| | A8 | B8 | C8 | a8 | b8 |
| Aqueous ethanol solution (40-50) | 45 | 45 | 45 | 45 | 45 |
| EDTA (0.1-0.2) | 0.15 | 0.15 | 0.15 | 0.15 | 0.15 |
| Dihydric alcohol additive (0.2-1) | 0.3 | 0.6 | 0.9 | 0.1 | 1.4 |
| Gelatin (5-20) | 13 | 13 | 13 | 13 | 13 |

Table 9 presents the experimental conditions in which the mixing ratio of only gelatin is varied while the mixing ratios of other components are fixed in the second embodiment of the present invention.

**[Table 9]**

| "Experiment 9" | Example | | | Comparative Example | |
|---|---|---|---|---|---|
| | A9 | B9 | C9 | a9 | b9 |
| Aqueous ethanol solution (40-50) | 45 | 45 | 45 | 45 | 45 |
| EDTA (0.1-0.2) | 0.15 | 0.15 | 0.15 | 0.15 | 0.15 |
| Dihydric alcohol additive (0.2-1) | 0.6 | 0.6 | 0.6 | 0.6 | 0.6 |
| Gelatin (5-20) | 6 | 13 | 19 | 3 | 23 |

In Tables 6 to 9, in Examples (A6 to A9, B6 to B9, and C6 to C9), the fixing ability of the jellified cell fixing means was favorable. On the other hand, in Comparative Examples (a6 to a9 and b6 to b9), the ability to fix the specimen inside the vial 10 was too strong or too weak. Therefore, in Comparative Examples, a problem (for example, specimen deformation) occurred in the process of taking out the specimen from the vial 10 and examining (for example, redissolving) the specimen.

In order to obtain the alcohol-based jelly composition for cell fixation according to the second embodiment of the present invention as well, experiments were conducted in addition to those presented in Tables 6 to 9, but the description of the other experiments has been omitted for convenience of explanation.

Meanwhile, in the second embodiment of the present invention, 5 to 20 parts by weight of any one or more of gelatin or a photocurable protein (gelatin methacryloly) may be substituted with 1 to 100 parts by weight of a natural cellulose polymer (for example, mecellose or hecellose) or 1 to 100 parts by weight of synthesized acrylic polymer beads (for example, methyl methacrylate-co-ethylene glycol dimethacrylate; MMA).

Here, with regard to 1 to 100 parts by weight of a natural cellulose polymer as well, an experiment was conducted in mixing ratio ranges of the natural cellulose polymer of less than 1 part by weight and more than 100 parts by weight as Comparative Examples while the mixing ratios of other components were fixed as in Tables 6 to 9, and as a result, the specimen fixing ability was poor in the region outside of 1 to 100 parts by weight.

In addition, with regard to 1 to 100 parts by weight of synthesized acrylic polymer beads as well, an experiment was conducted in mixing ratio ranges of the synthesized acrylic polymer beads of less than 1 part by weight and more than 100 parts by weight as Comparative Examples while the mixing ratios of other components were fixed as in Tables 6 to 9, and as a result, the specimen fixing ability was poor in the region outside of 1 to 100 parts by weight.

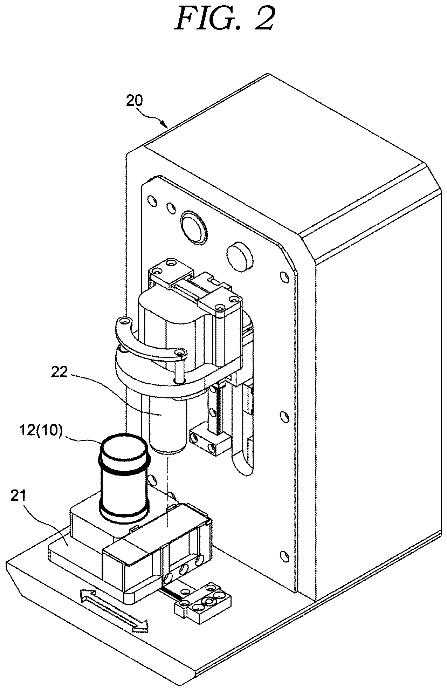

FIG. 2 is an exemplary view of a state in which a vial is mounted on a stirrer for jellification. Referring to FIG. 2, a stirrer for jellification 20 may be equipped as a method for jellifying the cell fixing means in the present invention.

First, the vial 10 is mounted on a table member 21 of the stirrer for jellification 20. In this state, when the stirrer for jellification 20 is operated, the table member 21 is positioned under an injection member 22, and the jelly composition is filled in the vial 10 as the jelly composition is dropped downward from the injection member 22.

The jelly composition in the vial 10 may be thoroughly mixed by operating the stirrer for jellification 20 and thus reciprocating the table member 21 in the direction of the arrow in FIG. 2.

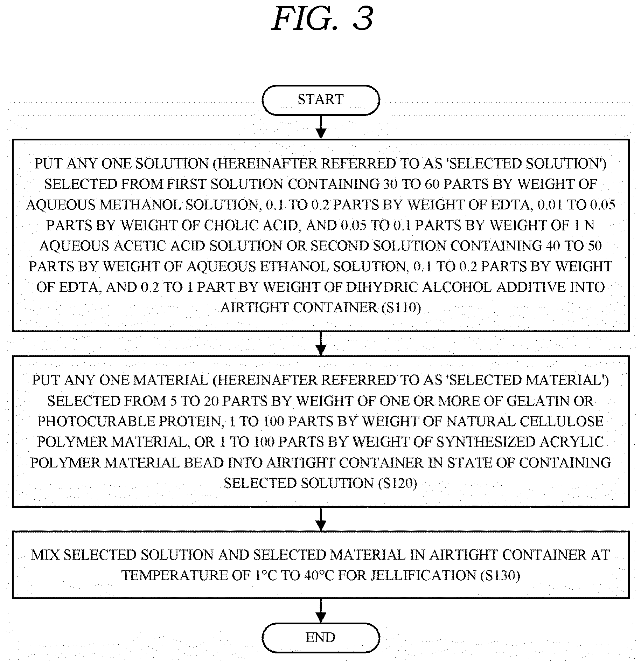

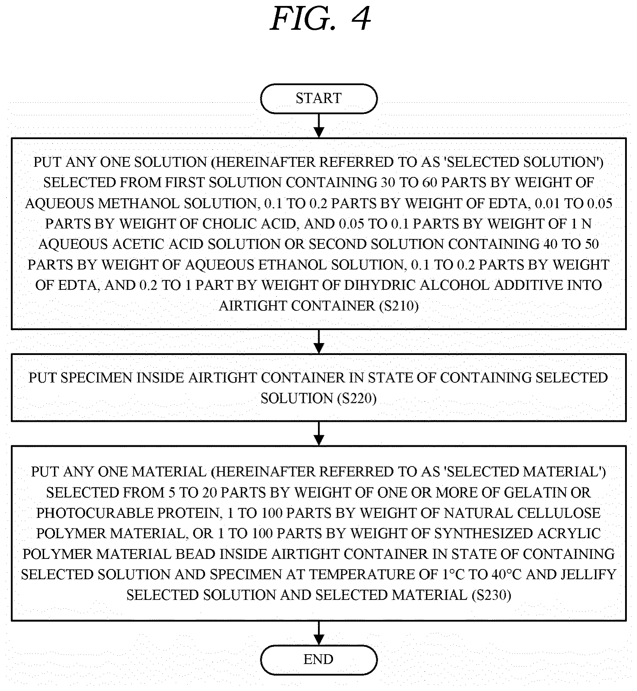

With reference to FIGS. 3 and 4, the process of jellifying the jelly composition in the present invention will be described in detail below.

FIG. 3 is a flow chart illustrating a jellification process using the alcohol-based jelly composition for cell fixation according to the first embodiment of the present invention.

Step S 110: First, any one solution (hereinafter referred to as 'selected solution') selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive is put into the vial 10.

At this time, the vial 10 is preferably disposed in the vertical lower portion of the injection member 22 in a state of being mounted on the table member 21 of the stirrer for jellification 20.

Step S120: Subsequently, any one material (hereinafter referred to as 'selected material') selected from 5 to 20 parts by weight of one or more of gelatin or a photocurable protein, 1 to 100 parts by weight of a natural cellulose polymer material, or 1 to 100 parts by weight of synthesized acrylic polymer material beads is put into the vial 10.

Step S130: Next, the cell fixing means is jellified by mixing the selected solution and selected material in the vial 10 at a temperature of 1°C to 40°C. At this time, it is preferable to thoroughly mix the selected solution and selected material in the vial 10 by adjusting the stirrer for jellification 20 so that the table member 21 on which the vial 10 is mounted moves left and right in the direction of the arrow in FIG. 2.

Here, when the temperature around the vial 10 is as low as less than 1°C during jellification, the gelatin, photocurable protein, natural cellulose polymer material, and synthesized acrylic polymer beads in the vial 10 do not dissolve, resulting in poor gelation or jellification of the cell fixing means.

When the temperature around the vial 10 is as high as more than 40°C during jellification, the alcohol-based components having low boiling points evaporate, thus changes in the physical properties of the entire composition are caused, and the specimen fixing ability of the cell fixing means is too strong or weak.

FIG. 4 is a flow chart illustrating a jellification process using the alcohol-based jelly composition for cell fixation according to the second embodiment of the present invention.

Step S210: First, any one solution (hereinafter referred to as 'selected solution') selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive is put into the vial 10.

At this time, the vial 10 is preferably disposed in the vertical lower portion of the injection member 22 in a state of being mounted on the table member 21 of the stirrer for jellification 20.

Step S220: Subsequently, a specimen is put inside the vial 10 in a state of being filled with the selected solution.

Step S230: Next, any one material (hereinafter referred to as 'selected material') selected from 5 to 20 parts by weight of one or more of gelatin or a photocurable protein, 1 to 100 parts by weight of a natural cellulose polymer material, or 1 to 100 parts by weight of synthesized acrylic polymer material beads is put inside the vial 10 in a state of containing the selected solution and the specimen at a temperature of 1°C to 40°C, and the selected solution and selected material as a cell fixing means are jellified.

At this time, the selected solution and selected material in the vial 10 may be thoroughly mixed by adjusting the stirrer for jellification 20 so that the table member 21 on which the vial 10 is mounted moves left and right in the direction of the arrow in FIG. 2.

Here as well, when the temperature around the vial 10 is as low as less than 1°C during jellification, the gelatin, photocurable protein, natural cellulose polymer material, and synthesized acrylic polymer beads in the vial 10 do not dissolve, resulting in poor gelation or jellification of the cell fixing means.

When the temperature around the vial 10 is as high as more than 40°C during jellification, the alcohol-based components having low boiling points evaporate, thus changes in the physical properties of the entire composition are caused, and the specimen fixing ability of the cell fixing means is too strong or weak.

## Claims

1. An alcohol-based jelly composition for cell fixation comprising 30 to 60 parts by weight of an aqueous methanol solution, 0.1 to 0.2 parts by weight of EDTA, 0.01 to 0.05 parts by weight of cholic acid, 0.05 to 0.1 parts by weight of a 1 N aqueous acetic acid solution, and 5 to 20 parts by weight of one or more of gelatin or a photocurable protein.

## Patentansprüche

1. Alkoholbasierende Gelzusammensetzung zur Zellfixierung, umfassend 30 bis 60 Gew.-Teile einer wässrigen Methanollösung, 0,1 bis 0,2 Gew.-Teile EDTA, 0,01 bis 0,05 Gew.-Teile Cholsäure, 0,05 bis 0,1 Gew.-Teile einer 1 N wässrigen Essigsäurelösung und 5 bis 20 Gew.-Teile einer oder mehrerer von Gelatine oder einem photohärtbaren Protein.

## Revendications

1. Composition de gelée à base d'alcool pour une fixation cellulaire comprenant 30 à 60 parties en poids d'une solution aqueuse de méthanol, 0,1 à 0,2 parties en poids d'EDTA, 0,01 à 0,05 parties en poids d'acide cholique, 0,05 à 0,1 parties en poids d'une solution aqueuse d'acide acétique 1 N, et 5 à 20 parties en poids d'une ou de plusieurs parmi la gélatine ou une protéine photodurcissable.
